# EUROPÄISCHE PATENTSCHRIFT

(11) **EP 1 152 767 B1**
(45) Veröffentlichungstag und Bekanntmachung des Hinweises auf die Patenterteilung: **16.06.2004**
(21) Anmeldenummer: 00912481.9
(22) Anmeldetag: 17.02.2000
(51) Int. Cl.: A61K 35/14, A61K 35/28, C12N 5/08, A61P 35/00

(54) **VERFAHREN ZUR HERSTELLUNG ANTITUMORALER TH1-ZELLEN**
METHOD FOR PRODUCING ANTI-TUMOR TH1 CELLS
PROCEDE DE PRODUCTION DE CELLULES TH1 ANTITUMORALES

(30) Priorität: 18.02.1999 DE 19906744
(43) Veröffentlichungstag der Anmeldung: 14.11.2001
(73) Patentinhaber: GSF-Forschungszentrum für Umwelt und Gesundheit GmbH, 85764 Neuherberg (DE); Röcken, Martin, 80337 München (DE); Egeter, Oliver, 80337 München (DE)
(72) Erfinder: RÖCKEN, Martin, Dermatologische Klinik der LMU, 80337 München (DE); EGETER, Oliver, Dermatologische Klinik der LMU, 80337 München (DE); MOCIKAT, Ralph, GSF Forschungsz. für U. und G.GmbH, 85764 Neuherberg (DE)
(74) Vertreter: Grättinger & Partner (GbR)
(86) Internationale Anmeldenummer: PCT/EP2000/001339
(87) Internationale Veröffentlichungsnummer: WO 2000/048614

(56) Entgegenhaltungen:
- WO-A-98/50527
- ZIMMERMANN S ET AL: "CpG oligodeoxynucleotides trigger protective and curative Th1 responses in lethal murine leishmaniasis." JOURNAL OF IMMUNOLOGY, (1998 APR 15) 160 (8) 3627-30. , XP002145844
- KUGE S ET AL: "Superantigen-induced human CD4+ helper/killer T cell phenomenon - Selective induction of Th1 helper/killer T cells and application to tumor immunotherapy" JOURNAL OF IMMUNOLOGY, Bd. 154, Nr. 4, 1995, Seiten 17777-1785, XP002145845 in der Anmeldung erwähnt
- OGASAWARA MASAHIRO ET AL: "Enhanced expression of HLA molecules and stimulation of autologous human tumor infiltrating lymphocytes following transduction of melanoma cells with gamma-interferon genes." CANCER RESEARCH, Bd. 53, Nr. 15, 1993, Seiten 3561-3568, XP002145846 ISSN: 0008-5472
- EGETER O ET AL: "Effective therapy of murine A20-tumors with A20 specific T helper 1 cells." FASEB JOURNAL, Bd. 13, Nr. 4 PART 1, 12. März 1999 (1999-03-12), Seite A297 XP002145847 ISSN: 0892-6638
- EGETER OLIVER ET AL: "Eradication of disseminated lymphomas with CpG-DNA activated T helper type 1 cells from nontransgenic mice." CANCER RESEARCH, Bd. 60, Nr. 6, 15. März 2000 (2000-03-15), Seiten 1515-1520, XP000938561 ISSN: 0008-5472

## Beschreibung

Die Erfindung betrifft ein Verfahren zur Herstellung von Interferon-γ (IFN-γ) produzierenden, antitumoralen Th1-Zellen.

Das Immunsystem der Säuger, einschließlich das des Menschen verfügt über eine Reihe von Strategien, um vor verschiedenen Tumoren zu schützen oder sogar sie zu bekämpfen. So ist es möglich, Immunantworten gegen Tumorantigene, das sind (Glyko-)Proteine, die speziell auf der Oberfläche oder im Inneren von Tumorzellen exprimiert werden, hervorzurufen. Immunantworten gegen diese Antigene können vor dem Etablieren von soliden Tumoren und Tumoren des hämatopoietischen Systems schützen. Aus der Literatur geht hervor, daß die meisten Immunantworten gegen Tumoren sowohl von den CD4+T-Helferzellen (Th) als auch den zytotoxischen CD8+T-Zellen (Tc) abhängig sind. Es gilt jedoch als anerkannt, daß insbesondere die zytotoxischen Tc für eine wirksame Tumorabwehr von Bedeutung sind. Die Rolle der Th wurde bisher in dieser Hinsicht nur wenig untersucht.

Es wurden zahlreiche Medikamente und Verfahren zur Immunisierung gegen verschiedene Tumortypen entwickelt. Als besonders wirksam in der Tumorprävention haben sich beispielsweise Immunisierungen mit den dendritischen antigenpräsentierenden Zellen (APC), die Koapplikation von proinflammatorischen Mediatoren, insbesondere Interleukin-(IL)-12 und die T-Zellaktivierung durch Modulation des CTLA4-Moleküls erwiesen. Weitere Strategien beinhalten Vakzinierungen mit Präparaten, die entweder tumorinfiltrierende T-Lymphozyten (TIL), lymphokinaktivierte Killerzellen (LAK) oder CD8+ T-Lymphozyten enthalten. Derartige Ansätze erwiesen sich jedoch als nur eingeschränkt wirksam.

Obgleich zahlreiche Immunisierungsprotokolle zur Tumorprävention beschrieben wurden, gibt es bisher kaum über das Immunsystem wirkende Medikamente, die bei bereits etablierten Tumoren wirksam sind. Das einzig wirklich wirksame Therapieprotokoll ist der adoptive Transfer allogener T-Lymphozyten in der Behandlung von Leukämien. Die Daten der letzten 20 Jahre belegen, daß IFN-γ produzierende T-Lymphozyten eine wichtige Rolle bei der zellulären Tumorabwehr spielen. Bisher wurden vor allem Tumorimmunantworten untersucht, die sich insbesondere gegen Antigene richten, die über die Haupthistokompatibilitäts-Antigene der Klasse I (MHC I) an Tc präsentiert werden. MHC-Klasse II-restringierte Th werden als Regulatorzellen angesehen. Ihre Gegenwart scheint für eine effiziente Tumorprävention wichtig. Trotzdem kennt man weder ihre genaue Rolle bei der antitumoralen Immunantwort, noch sind, von wenigen Ausnahmen abgesehen, ihre Zielpeptide bekannt (Immunity 95: 2, 45-57). Wegen dieser Schwierigkeiten wurden sie bisher nicht als Medikament im Sinne von Immuntherapien gegen spezifische Tumor entwickelt.

In WO99/51720 ist ein (nachveröffentlichtes) sehr spezielles Verfahren beschrieben, mit dem Th1-Zellen generiert werden können. Es erlaubt aber nicht, wirksam CD2-positive Zellen oder T-Zellen zu entwickeln, die spezifisch ein Antigen, wie z.B. ein Tumor-Antigen erkennen.

Ein bekanntes Verfahren (Kuge et al 1995 J. Immunol 154(4), 1777-85) beschreibt, daß Th1-Lymphozyten Tumorzellen in der.Kulturschale lysieren können. Hinweise auf eine in vivo-Therapie sind dieser Schrift jedoch nicht entnehmbar. Auch läßt sich dieser Ansatz nicht direkt auf die in vivo-Situation übertragen.

Der vorliegenden Erfindung liegt die Aufgabe zugrunde, ein Verfahren zur Herstellung eines Präparats zur Prophylaxe und Therapie von Tumorerkrankungen bereitzustellen. Insbesondere geht es darum, spezifisch tumorreaktive Zellen in vitro zu entwickeln, die in vivo gegen Tumoren wirksam sind. Ziel einer jeden Tumortherapie ist es, den Tumor soweit als möglich zu zerstören und dabei alle anderen Zellen des Körpers zu schonen. Die herkömmlichen Therapien erreichen dies nur unzureichend. Aus diesem Grunde hat man versucht, spezifische Tumortherapien zu entwickeln. Der einzige Therapieansatz, der nach heutigem Verständnis tumorspezifisch ist, ist eine Immuntherapie, da die Lymphozyten dank ihres Antigenrezeptors nur jene Zielzellen erkennen, gegen die sie aktiviert wurden.

Wegen ihrer Fähigkeit, Tumorzellen direkt zu töten, setzt die Forschung von heute ganz überwiesend auf eine antitumorale Therapie mittels tumorspezifischer Tc. Von Autoimmunkrankheiten her ist aber bekannt, daß nicht Tc, sondern Th Autoimmunkrankheiten besonders dramatisch auslösen (Racke et al. 1994). Besonders schwere, oftmals akut tödlich verlaufende Formen von Autoimmunkrankheiten werden durch den adoptiven Transfer von IFN-γ produzierenden Th, die auch Th1 genannt werden, ausgelöst. Da diese adoptiv transferierten Th1 zwar das Zielorgan zerstören, dabei aber alle anderen Gewebe vollständig verschonen, sollte sich der adoptive Transfer tumorspezifischer Th1 besonders gut zur Tumortherapie eignen.

Das erfindungsgemäß hergestellte Präparat soll vor einer möglichen Tumorentwicklung schützen und insbesondere bei bereits etablierten Tumoren ebenfalls wirksam sein. Ferner soll das erfindungsgemäß hergestellte Präparat dem jeweils zu bekämpfenden Tumor angepaßt werden können. Das Präparat soll die natürlichen Mechanismen der Tumorabwehr nachahmen bzw. verstärken und somit keine oder nur geringe Nebenwirkungen besitzen.

Gelöst wird diese Aufgabe durch ein Verfahren zur Herstellung eines pharmazeutischen Präparats, das tumorspezifische Th enthält, die durch ein Th1-ähnliches Zytokinmuster charakterisiert sind, sich also durch eine hohe IFN-γ-Produktion P1 (100 ≤ P1 ≤ 100000 U/ml) und eine niedrige IL-4-Produktion P2 (P2 ≤ 1000 U/ml) auszeichnen. Von besonderer Bedeutung ist dabei das Verhältnis P1:P2, welches in der Größenordnung 10:1 oder größer liegen soll. Die T-Lymphozyten werden zusammen mit pharmazeutisch verträglichen Exzipientien und Hilfsstoffen verabreicht. Es wird ein Verfahren zur Herstellung von tumorspezifischen Th1-Zellen etabliert. Es sind Zellen, die den Tumor oder tumorassoziierte Antigene (Aminosäuren/ Eiweißsequenzen) erkennen und gegen diese reagieren. Sie zeigen aber kaum oder keine Reaktivität gegenüber anderen Antigenen, die im Körper effizient präsentiert werden. Die Erfindung betrifft, auch die Verwendung dieser Th1 zur Herstellung eines Medikaments für die Prophylaxe und/oder Therapie von soliden Tumoren und Tumoren des hämatopoietischen Systems.

In Anlehnung an die Experimente zur Pathogenese von Autoimmunkrankheiten wurde gefunden, daß Th, insbesondere Th1 und Th1-ähnliche Zellen, in der Tumorabwehr eine signifikante Bedeutung besitzen. Durch Züchten von Th1, die speziell (Glyko-)Proteine des zu bekämpfenden Tumors erkennen, können wirksame Medikamente zur Prophylaxe/Bekämpfung des Tumors hergestellt werden. Präparate, die diese Zellen enthalten, werden dem Organismus auf parenteralem Weg, meist intraperitoneal, intravenös oder subkutan verabreicht. Die verabreichten Th1 aktivieren bzw. verstärken die gegen den Tumor gerichtete Immunantwort entweder durch direkte Induktion von Zelltod oder durch Produktion ihrer spezifischen Zytokine. Diese können entweder für den Tumor direkt zytotoxisch sein oder dazu führen, daß für den Tumor toxische Effektorzellen, Tc oder Makrophagen, rekrutiert und aktiviert werden. Dadurch haben sie entscheidenden Anteil an der immunvermittelten Remission bzw. Regression des Tumors.

Erfindungsgemäß können Th1-Zellen gegen beliebige Tumorarten produziert werden. Bevorzugt ist die Produktion von Th1 gegen Lymphome wirksam, aber auch solide Tumoren sind wichtige Zielstrukturen. Mit dem erfindungsgemäß hergestellten Präparat können daher die entspechenden Tumorarten bekämpft werden. Die Art und Posologie der Verabreichung werden von einem Arzt entsprechend der Art und Schwere des zu behandelnden Tumors, dem Zustand des Patienten und der Therapiedauer bestimmt. Das erfindungsgemäß hergestellte Präparat in einer Dosierung von 1x10⁶-100-1000x10⁶ Zellen/kg Körpergewicht in einer einmaligen Dosis oder wiederholt verabreicht. Das Präparat kann sowohl Menschen als auch Tieren verabreicht werden.

Die erfindungsgemäß hergestellten Th1-Zellen können vor hämatopoietischen Tumoren schützen; sie eignen sich auch zur Behandlung etablierter Lymphome und es können mit derartigen Th1-Zellen auch solide Tumoren zumindest kontrolliert werden.

Das erfindungsmäßige Verfahren erlaubt ferner, tumorspezifische Th1-Zellen auch aus Tieren zu gewinnen, die bereits an einem fortgeschrittenen Tumor erkrankt sind. Dabei ist es möglich, aus der Vielzahl der unterschiedlichen Immunzellen genau die wenigen Familien von T-Lymphozyten zum Wachsen zu bringen, die entweder spezifisch oder vor allem den Tumor erkennen. Durch den adoptiven Transfer tumorspezifischer Th1-zellen können sehr schnell wirksame antitumorale Immunantworten aufgebaut werden, die dann auch in der Therapie sehr schnell wirksam und dabei sicher - d.h. weitgehend frei von Nebenwirkungen sind.

Um die Rolle von tumorreaktiven MHC-Klasse II restringierten CD4+Th näher zu untersuchen, war es nötig, ein Kultursystem zu entwickeln, das es erlaubt, tumorspezifische Th1 zu generieren. MHC-Klasse II restringierte Tumorpeptide sind bisher kaum bekannt. Aus diesem Grund wurde ein System entwickelt, das es erlaubt, tumorspezifische Th1 zu etablieren, ohne spezifische Tumorpeptide zu kennen. Durch Bestrahlung oder Chemotherapeutika wie Mitomycin C in ihrer Proliferation gehemmte Tumorzellen werden zusammen mit autologen/syngenen APC und Th in vitro kultiviert. Aus Gründen der Praktibilität wurden die Untersuchungen bisher an einem B-Zell-Lymphom durchgeführt, das seine eigenen Tumorantigene über MHC II präsentieren kann. Aus Mäusen, die das A20-B-Zell-Lymphom bereits tragen, oder aber auch aus Mäusen, die gegen dieses Lymphom immunisiert wurden, wurden CD4+Th gewonnen. Diese Th wurden dann in vitro mit der unten beschriebenen Methode so kultiviert, daß tumorspezifische Th1 entstehen, Th, die große Mengen an IFN-γ, aber wenig oder kaum IL-4 produzieren. Gemessen mit einem handelsüblichen ELISA-Test lagen wirksame IFN-γ-Spiegel zwischen 10² und 10⁵ U/ml; gemessen mit CT4S-Zellen mußte IL-4 unter 1000 U/ml liegen. Wieder war wichtig, daß die Ratio von IFN-γ zu IL-4 ≥ 10:1 betrug. Diese tumorspezifischen Th1 wurden dann zur Tumortherapie verwendet.

### Material-Herstellung tumorspezifischer Th1

Murine A20-Zellen (ATCC-TIB-208) wurden in RPMI1640, das 10 % FCS und 50 mM β-Mercaptoethanol enthielt, bei 37°C in wassergesättigter Atmosphäre und 5 % CO₂ kultiviert. Vor dem Einsatz zur in vitro Kultur mit den Th wurden die Tumorzellen mit 65 Gray bestrahlt.

Th (> 95% Reinheit) wurden durch negative Selektion unter Verwendung von Biotex-T-Zellsäuren (Tebu, Frankfurt) gewonnen. Andere Verfahren zur Gewinnung von Th mittels positiver oder negativer Anreicherung sind in gleicher Weise geeignet. Diese Th wurden in vitro in Dulbeccos MEM (Kulturmedium), das durch 10% FCS, Penicillin und Streptomycin, 50 µM β-Mercaptoethanol und MEM-Aminosäuren angereichert worden ist, bei 37°C in wassergesättigter Atmosphäre und 7,5 % CO₂ kultiviert. Auch andere Medien erlauben, Th Linien zu etablieren, einige davon ohne die Verwendung von Serum.

APC wurden durch Behandlung von BALB/c-Milzzellen mit Anti-CD4- und Anti-CD8-Antikörper und Komplement (Behring-Werke, Marburg) angereichert. Vor dem Einsatz zur in vitro Kultur von Th wurden sie mit 30 Gray bestrahlt.

Phosphothioatmodifiziertes CpG-Dinucleotidhaltiges Oligonucleotid (CpG-ODN 1668) wurde von MWG (München) bezogen (Sequenz: 5'-TCC ATG ACG TTC CTG ATG CT-3'). Anti-IL-4-Antikörper stammen von dem Klon 11B11, humanes rIL-2 wurde im Handel bezogen. Derartige Antikörper sind auch gegen das IL-4 des Menschen entwickelt worden; weiterhin lassen sich Th1 auch durch die Anwendung von solublem IL-4 Rezeptormolekül herstellen (Breit et al. 1996, Eur.J.Immunol. 26, 1860-1865).

### Beispiel für die Herstellung der tumorspezifischen Th1

### Tag 1

### 1. Stimulation

0,2 x 10⁶ Th + bestrahlte Tumorzellen (0,2 x 10⁶ A20) + 0,3 x 10⁶ APC + anti-IL-4 Antikörper (z.B. 10 µg/ml 11B11) +/- immunstimulatorische Oligonukleotide (z.B. 0,2 µM CpG-ODN 1668) + IL-2 in einer 96-Loch-Rundboden-Kulturplatten, 200 µl Volumen, in Kultur gegeben.

### Tag 2

Wechsel von Medium, anti-IL-4 Antikörper, Oligonukleotid und IL-2.

### Tag 4

Die Zellsuspension wird in eine 24-Loch-Kulturplatte überführt und mit einem IL-2-haltigen Medium ernährt.

### Tag 5 - 10

Alle 2 Tage (Tag 7, Tag 9) erfolgt ein Mediumwechsel und die Ernährung der Zellen mit einem IL-2-haltigen Medium. Je nach Zelldichte wird die Kultur auf weitere Kulturschalen verteilt.

### Tag 11

### 2. Stimulation der Zellen

0,1 x 10⁶ Th + 0,2 x 10⁶ bestrahlten A20 + 0,3 x 10⁶ APC + anti-IL-4 Antikörper +/- immunstimulatorische Oligonukleotide in einer 96-Loch-Rundboden-Kulturplatte, 200 µl Volumen, in Kultur gegeben.

### Tag 12

Wechsel von Medium, anti-IL-4 Antikörper, Oligonukleotid und IL-2.

### Tag 14

Die Zellsuspension wird in eine 24-Loch-Kulturplatte überführt und mit einem IL-2-haltigen Medium ernährt.

### Tag 15 - 20

Alle 2 Tage (Tag 17, Tag 19) erfolgt ein Mediumwechsel und die Ernährung der Zellen mit IL-2-haltigen Medium. Je nach Zelldichte wird die Kultur auf weitere Kulturschalen aufgeteilt.

### Tag 21

### 3. Stimulation

Je nach Bedarf können die Th alle 10-14 Tage, so an Tag 21 noch ein drittes Mal und öfter, entsprechend dem Protokoll für die 2. Stimulation (Tag 11-20) restimuliert und weiter expandiert werden. Alternativ werden sie für die Therapie mittels adoptivem Transfer genutzt.

Bei Kontrolle exprimieren diese Linien Oberflächenantigene, die aktivierte Th charakterisieren. Die so generierten Th-Linien sind:
(1) spezifisch für den Tumor, hier das A20-Lymphom und
(2) T-Zell-Linien von Th1 Typ (Th1-ähnliche Zellen).

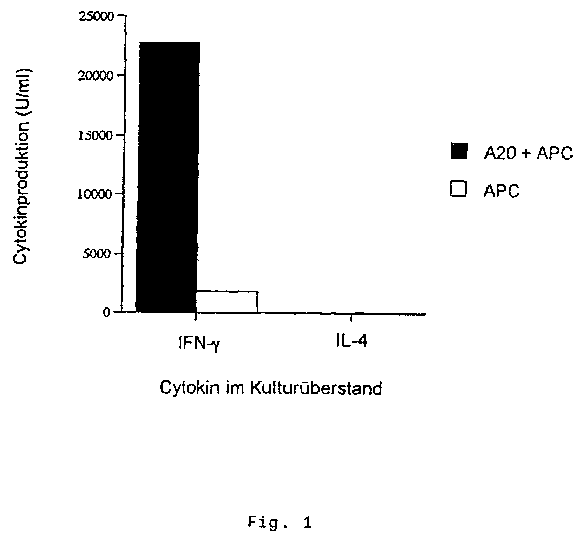

Werden sie in vitro durch syngene Milzzellen stimuliert, die über ihre MHC Klasse II Moleküle die Tumorpeptide des A20-Tumors präsentieren, produzieren sie viel IFN-γ und wenig oder kein IL-4 (vgl. Produktionsangaben auf S. 6 und Fig. 1). Werden die Th jedoch mit syngenen Milzzellen kultiviert, die keine Tumorantigene oder Antigene eines anderen Tumors, des MCP11-B-Zell-Lymphoms, präsentieren, prodzieren sie keine, oder nur geringe Mengen an Zytokinen. Diese Zellen stellen pharmazeutische Präparate dar, die gezielt zur Therapie des A20-Lymphoms verwendet werden können.

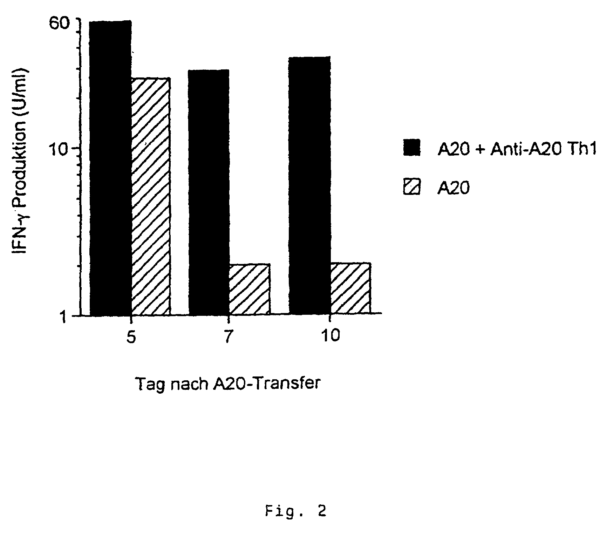

Zunächst wurde die Auswirkung des adoptiven Transfers von A20-spezifischen Th1 auf die Immunantwort von Balb/c-Mäusen gegen den A20-Tumor untersucht. Dazu wurden Balb/c-Mäusen gleichzeitig 0,5 x 10⁶ A20-Zellen und 0,5 x 10⁶. A20-spezifische Th1 intraperitoneal injiziert. Nach 5, 7 und 10 Tagen wurden die Milzzellen aus den Mäusen isoliert und in vitro mit dem Tumor, hier dem 0,2 x 10⁶ A20-Tumor, der durch syngene APC präsentiert wurde, stimuliert. T Lymphozyten von Mäusen, denen Tumorzellen verabreicht worden waren, produzierten IFN-γ nur dann, wenn sie innerhalb von 5 Tagen nach Tumorapplikation isoliert wurden. Im Gegensatz dazu produzierten T-Lymphozyten von Mäusen, die sowohl A20-Tumoren als auch A20-spezifische Th1 injiziert bekamen, auch dann noch IFN-γ, wenn sie mehr als 10 Tage nach dem Tumortransfer durch syngene APC mit tumorspezifischen Antigenen stimuliert wurden (Fig. 2). Durch den adoptiven Transfer von A20-spezifischen Th1 wurde so eine prolongierte Th1 Immunantwort gegen den A20-Tumor induziert.

### Tumorprävention mit tumorreaktiven Th1

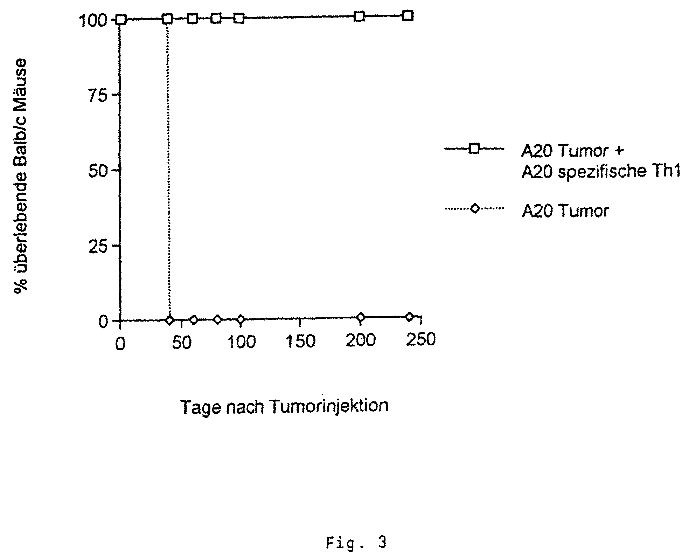

In Tumorpräventionsexperimenten wurden den Balb/c-Mäusen direkt nacheinander 0,5 x 10⁶ A20-Tumorzellen und 0,5 x 10⁶ A20-spezifische Th1 intraperitoneal injiziert. Durch den adoptiven Transfer der Th1-Linien wurde die Überlebensrate der Mäuse erheblich verlängert (Fig. 3). Je nach Experiment waren zwischen 70% und 100% aller Mäuse völlig geheilt. Dies war insofern erstaunlich, als der Tumor als sehr aggressiv gilt und viele herkömmliche Immuntherapien, die sonst wirksam eine Tumorprävention erlauben, bei diesem Tumor versagen. Ein zweites wichtiges Ergebnis war, daß ein adoptiver Transfer dieser Th1-Linien zwar vor der Tumorentwicklung schützt, daß er aber nicht zu Autoimmunerkrankungen führt: die Tiere können unter dem Schutz so lange leben, daß sie eines natürlichen Todes sterben. Auch nach einem Jahr weisen die Tiere weder klinisch noch in der Autopsie Anzeichen für Tumoren oder Autoimmunerkrankungen auf. Selbst wenn die Tiere immunsupprimiert werden, treten zu einem späteren Zeitpunkt keine Tumoren mehr auf.

### Tumortherapie mit tumorreaktiven Th1

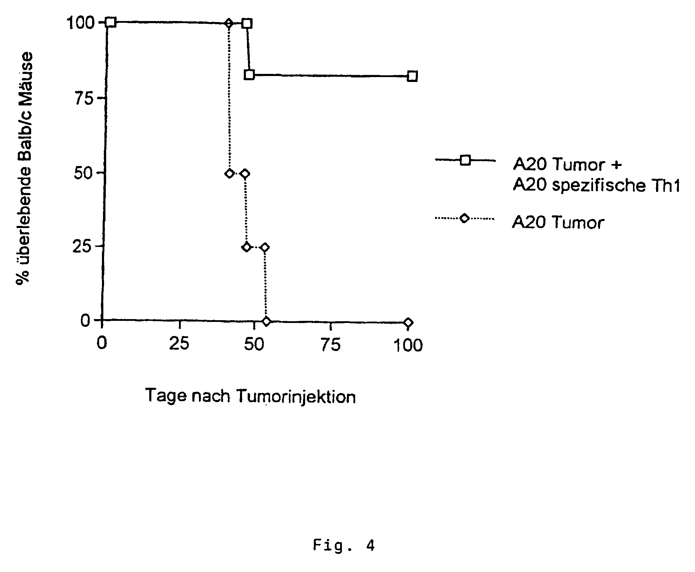

Aufbauend auf diesen Ergebnissen wurden dann die Th1 genutzt, um bereits etablierte Tumoren zu behandeln. 0,5 x 10⁶ A20-Tumorzellen wurden intravenös injiziert. An Tag 7 nach dem Transfer, einem Zeitpunkt, zu dem sich bereits große Mengen an Tumor in den lymphoiden Organen nachweisen lassen, erhielten die Tiere wiederum eine intravenöse Vakzinierung von 0,5 x 10⁶ A20-spezifische Th1-Linien, also etwa 25 x 10⁶ Th1-Zellen pro kg Körpergewicht. Die Daten aus fünf Experimenten ergaben, daß (1) bei fast allen Tieren das Tumorwachstum erheblich verzögert wurde und (2) bis 70 % der Tiere durch den Transfer der Th1 als geheilt angesehen wurden (Fig. 4). Die Therapie war noch zu einem Zeitpunkt wirksam, zu dem Immuntherapien bisher nicht beschrieben wurden und zu dem die Tumorlast der Milz etwa 100-fach größer war als bei den bisher beschriebenen Immunisierungsprotokollen.

Somit zeigen die Daten in Fig. 2, 3 und 4, daß adoptiv transferierte Th1 sehr wirksame Immunantworten gegen Tumoren hervorrufen können. Erstmals wurde erfindungsgemäß gezeigt, daß
1) Th1 auch sehr effizient und sicher nicht nur in der Prävention sondern auch in der Tumortherapie eingesetzt werden können.
2) Die Therapie auch in immunkompetenten Tieren funktioniert.
3) Es ist davon auszugehen, daß mit der hier beschriebenen Methode auch Th1 gegen Zellen von anderen, insbesondere soliden Tumoren induziert werden können, da die Präsentation der Antigene vorwiegend über autologe/syngene APC (in diesem Ansatz Milzzellen) erfolgt. Die Methode erlaubt somit wirksame Th1 auch für eine Immunantwort gegen solide Tumoren, die kein MHC-II exprimieren, zu generieren.

Die beigefügten Figuren erläutern die Erfindung näher.

Die Fig. 1 zeigt die Produktion von IFN-γ und IL-4 durch A20-spezifische Th1-Zell-Linien nach In Vitro-Stimulation. Die A20-spezifische IFN-γ-Produktion einer CD4+T-Zell-Linie wurde mit CpG ODN und anti-IL-4-Antikörpern gegen das Lymphom innerhalb von zwei Wochen in vitro generiert. Dabei wurden CD4+-T-Zellen (Th) aus Balb/c-Mäusen isoliert, die durch einen A20-Tumor immunisiert worden waren. Die gewonnenen Th wurden in vitro in Gegenwart von syngenen antigenpräsentierenden Zellen (APC), A20-Tumorzellen, Anti-IL-4-Antikörper, IL-2 und CpG-ODN 1668 stimuliert und über 10 Tage expandiert. An Tag 11 wurden die kultivierten Th1 in Gegenwart von APC alleine oder zusätzlichen A20-Zellen für 24 Stunden restimuliert. Aus dem Kulturüberstand wurde der Gehalt an IL-4 und IFN-γ bestimmt.

Die Fig. 2 zeigt die Verlängerung einer A20-spezifischen Immunantwort durch adoptiven Transfer von A20-spezifischen Th1. Durch den adoptiven Transfer von A20-spezifischen Th1 wird die tumorspezifische Immunantwort gegen ein A20-B-Zell-Lymphom deutlich verlängert. Dabei wurden Balb/c-Mäuse durch intravenöse Injektion von 0,5 Millionen A20 Tumorzellen alleine oder zusammen mit 0,5 Millionen A20-spezifischen Th1 immunisiert. An d5, 7, 10 wurden die Milzen der Mäuse präpariert und 1 Million Zellen in vitro ohne oder mit bestrahlten A20 für 48 Stunden bei 37°C inkubiert. Aus dem Kulturüberstand wurde dann IFN-γ bestimmt.

Die Fig. 3 zeigt die Prävention eines A20 B-Zell-Lymphoms durch adoptiven Transfer von A20-spezifischen Th1. Es kommt zu schnellem Sterben von BALB/c-Mäusen nach adoptivem Transfer des A20-Lymphoms (Tumor) und zu langfristigem Überleben von BALB/c-Mäusen nach Gabe des A20-Lymphoms und gleichzeitigem, adoptivem Transfer von A20-spezifischen Th1. Dabei wurden Balb/c-Mäusen 0,5 Millionen A20-Tumorzellen alleine oder gleichzeitig mit 0,5 Millionen A20-spezifischen Th1 intravenös injiziert und der Therapieverlauf wurde visuell verfolgt.

Die Fig. 4 zeigt die Therapie eines etablierten A20 B-Zell-Lymphoms durch adoptiven Transfer von A20-spezifischen Th1. Es kommt zu schnellem Sterben von BALB/c-Mäusen nach adoptivem Transfer des A20-Lymphoms und zu Langzeitheilung bei bis zu ≥ 80 % der BALB/c-Mäuse, wenn sieben Tage nach Gabe des A20-Lymphoms A20-spezifische Th1 adoptiv transferiert wurden. Zu diesem Zeitpunkt ist die Tumorbelastung 100-fach höher als bei der bisher erfolgreichsten Therapie mit Zytokin transferierten Tumorzellen. Dabei wurden Balb/c-Mäusen 0,5 Millionen A20-Tumorzellen in 500 µl PBS intravenös injiziert. Sieben Tage nach A20-Injektion (100 CFU/Milz) wurden den Mäusen 500 µl PBS alleine oder 0,5 Millionen A20-spezifische Th1 in 500 µl PBS intravenös injiziert und der Therapieverlauf visuell verfolgt.

## Patentansprüche

1. Verfahren zur Herstellung eines pharmazeutischen Präparats, umfassend tumorspezifische Th-Zellen mit einem Th-1-ähnlichen Zytokinmuster, die eine hohe Produktion von Interferon-γ von wenigstens 100 U/ml aufweisen und mit höchstens 1000 U/ml wenig oder kein Interleukin-4 produzieren, wobei das Verhältnis der Interferon-γ-Produktion zur Interleukin-4-Produktion wenigstens 10:1 beträgt, aus Tumorzellen, zusammen mit pharmazeutisch verträglichen Exzipientien und Hilfsstoffen, wobei durch Bestrahlung oder Chemotherapeutika wie Mitomycin in ihrer Proliferation gehemmte Tumorzellen zusammen mit autologen/syngenen APC (Antigenpräsentierenden Zellen) und Th (CD4+T-Helferzellen) in vitro kultiviert werden.

## Claims

1. Process for producing a pharmaceutical preparation, comprising tumor-specific Th cells with a cytokine-pattern similiar to Th-1, which demonstrate a high production of interferon-γ of at least 100 U/ml and produce, with at most 1000 U/ml, little or no interleukin-4, where the ratio of the interferon-γ production to the interleukin-4 production is at least 10:1, from tumor cells , together with pharmaceutically compatible excipients and processing agents, whereby tumor cells inhibited in their proliferation by radiation or chemotherapeutics such as Mitomycin are cultured in vitro together with autologous/syngeneic antigen presenting cells (APC) and Th (CD4+T helper cells).

## Revendications

1. Procédé de production d'un produit pharmaceutique comprenant des cellules Th spécifiques d'une tumeur cible, avec un phénotype de cytokine semblable au Th-1 qui présentent un haut taux de production d'interféron γ d'au moins 100 U/ml et peu ou pas d'interleukin 4 - 1.000 U/ml maximum, le rapport de l'interféron γ à l'interleukin 4 étant au minimum de 10 à 1, de cellules tumorales, avec des excipients et adjuvants pharmaceutiquement compatibles, les cellules tumorales inhibées dans leur prolifération par irradiation ou par des drogues chimiothérapeutiques du type mitomycine étant mises en culture in vitro avec des cellules qui présentent l'antigène (APC) autologues/syngéniques et des cellules T helper (CD4+T).
